(19) Europäisches Patentamt
European Patent Office
Office européen des brevets

(11) **EP 2 334 229 B2**

(12) **NEW EUROPEAN PATENT SPECIFICATION**
After opposition procedure

(45) Date of publication and mention
of the opposition decision:
**24.11.2021 Bulletin 2021/47**

(45) Mention of the grant of the patent:
**14.08.2013 Bulletin 2013/33**

(21) Application number: **09773783.7**

(22) Date of filing: **30.06.2009**

(51) Int Cl.:
**A61B 5/021** *(2006.01)*  **A61B 5/022** *(2006.01)*

(86) International application number:
**PCT/NL2009/050385**

(87) International publication number:
**WO 2010/002250 (07.01.2010 Gazette 2010/01)**

(54) **EVALUATE AORTIC BLOOD PRESSURE WAVEFORM USING AN ADAPTIVE PERIPHERAL PRESSURE TRANSFER FUNCTION.**

BEWERTUNG EINER AORTA-BLUTDRUCK-WELLENFORM MIT EINER ADAPTIVEN ÜBERTRAGUNGSFUNKTION DES PERIPHERISCHEN DRUCKS

ÉVALUATION D'UNE FORME D'ONDE DE PRESSION AORTIQUE EN UTILISANT UNE FONCTION DE TRANSFERT ADAPTATIVE DE PRESSION PÉRIPHÉRIQUE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.06.2008 EP 08159357**

(43) Date of publication of application:
**22.06.2011 Bulletin 2011/25**

(73) Proprietor: **Bmeye B.V.**
**1101 BE Amsterdam (NL)**

(72) Inventor: **STOK, Wim Johan**
**NL-1357 JG Almere (NL)**

(74) Representative: **Eisenführ Speiser**
**Patentanwälte Rechtsanwälte PartGmbB**
**Postfach 31 02 60**
**80102 München (DE)**

(56) References cited:
EP-A1- 0 765 630    WO-A2-01/78599
US-A- 5 265 011    US-B1- 6 647 287

- **SUGIMACHI MASARU ET AL: "A new model-based method of reconstructing central aortic pressure from peripheral arterial pressure", JAPANESE JOURNAL OF PHYSIOLOGY., CENTER FOR ACADEMIC PUBLICATIONS JAPAN., XX, vol. 51, no. 2, 1 April 2001 (2001-04-01), pages 217-222, XX ISSN: 0021-521X, DOI: 10.2170/jjphysiol.51.217**
- **Lasance, Wessling, K.H., Ascoop C.A.: "Peripheral Pulse Contour Analysis in Determining Stroke Volume, Progress Report 5, Inst. Med. Phys., Da Costakade 45, Utrecht, Netherlands, 1976**
- **Stok Wim J. et al: "Changes in finger-aorta pressure transfer function during and after exercise", Journal of Applied Physiology, vol. 101, no. 4, 2006, pages 1207-1214,**
- **B.E. Westerhof: "Journal of Applied Physiology, chapter 9", (Dissertation) Amsterdam, University of Amsterdam, 2005, pages 141-148,**
- **Costanzo L.: "INTEGRATIVE FUNCTIONS OF THE CARDIOVASCULAR SYSTEM", Physiology 3rd Ed., Chapter 4, 2006, pages 171-173,**
- **Plowman S.A. et al: "Cardiovascular Responses to Exercise", Exercise Physiology for Health, Fitness, and Performance, 2nd edition, 2003, Chapter 13, 2003, pages 354-356,**
- **WESSELING K H. et al: "COMPUTATION OF AORTIC FLOW FROM PRESSURE IN HUMANS USING A NONLINEAR, THREE-ELEMENT MODEL", JOURNAL OF APPLIED PHYSIOLOGY, AMERICAN PHYSIOLOGICAL SOCIETY, US, vol. 74, no. 5, 1 January 1993 (1993-01-01), pages 2566-2573, US ISSN: 8750-7587**
- **Guelen et al: "Finometer, finger pressure measurements with the possibility to reconstruct brachial pressure", Blood Press Monit., vol. 8, 2003, pages 27-30,**
- **Specification, Finometer; date stamp: Nov 25 2002; http://web.archive.org/web/20030217000427/ , http://finapres.com/index.php?pid=4230**

EP 2 334 229 B2

**Description**

FIELD

The invention relates to a method for reconstructing an aortic blood pressure waveform from a peripheral blood pressure waveform. The invention further relates to a device and a computer program product for reconstructing an aortic blood pressure from a peripheral blood pressure waveform. In particular, the invention relates to a method for reconstructing an aortic blood pressure waveform from a peripheral blood pressure waveform during exercise.

BACKGROUND OF THE INVENTION

The relevance of arterial blood pressure changes during exercise is well established and it is an independent predictor of cardiovascular morbidity and mortality. Peripheral arterial blood pressure can, for example, be measured during exercise with per se known systems which may be utilizing a suitable finger cuff or an applanation tonometry sensor. However, changes in peripheral pressure during exercise are not fully representative for changes in aortic pressure. Especially systolic pressure measured at a peripheral artery may be overestimated to a different extent between subjects and at increasing workloads.

The transfer of a blood pressure waveform along a vascular tree is usually characterized by a pressure transfer function (TF) in the frequency domain. The inverse of this TF may be used for the reconstruction of the central pressure from a pressure wave measured at a peripheral site. At rest and at low exercise loads, acceptable results for reconstructed aortic blood pressure waveform may be obtained using a fixed TF, as known from Stok et al, "Changes in finger-aorta pressure transfer function during and after exercise", J Appl Physiology 101: 1207 - 1214, 2006 and from Sharman et al "Validation of a generalized transfer function to noninvasively derived central blood pressure during exercise", Hypertension 46: 1203 - 1208, 2006.

During leg exercise the aortic-to-peripheral pressure transfer function may change. Therefore, methods for determining aortic blood pressure which do not take such change into account may be inaccurate. In principle, during exercise, two major changes occur in the hemodynamic status of a subject: both heart rate and mean blood pressure increase. The pressure transfer function used to describe waveform changes from aortic path to a periphery, expressed in the frequency domain, comprises a characteristic resonance peak frequency. Heart rate may have no direct influence on pressure transfer function, whereas an increase in mean blood pressure may change vascular diameter according to the compliance curve of the vessel, coupled with an increase in elastic modulus. This may have a direct effect on travel time over the arteries and on the transfer function, whereby travel time may be reduced and the resonance peak of the pressure transfer function may be shifted to higher frequencies. However, during leg exercise more complicated effects may occur due to vasoconstriction in the arm as upper arm flow may not undergo substantial changes despite a large increase in cardiac output. This may alter the properties of the large conduit arteries as well, but may also change the peripheral reflection coefficient of the vessels under study.

Due to the increase in heart rate (HR) during moderate to high workloads harmonics of the pressure wave may also be shifted to higher frequencies. The final result on aortic pulse pressure reconstruction with a generalized TF becomes rather unpredictable. These effects may reduce the applicability of a generalized transfer function (genTF). It may not be suitable to apply a generic, not individualized transfer function for determination of aortic blood pressure waveform from peripheral blood pressure waveform, particularly during exercise of the person.

An embodiment of a method for reconstructing aortic blood pressure waveform from peripheral blood pressure waveform is known from US 6, 647, 287. In the known method a model for such reconstructing is adjustable. Accordingly, aortic blood pressure waveforms are reconstructed from peripheral blood pressure waveform data using mathematical models. The models may combine analytical models of pulse wave propagation in the cardio-vascular system with empirical models derived from measurements taken from patients. When used to reconstruct the aortic pressure of a given subject, the models are adjusted to the subject's physiological state, for example, based upon ECG and other measurements.

It is a disadvantage of the known method for reconstructing aortic blood pressure that a considerable calculus and extensive real-time measurements are required for enabling due adjustment of used models for taking into consideration a physiological state of the patient.

SUMMARY OF THE INVENTION

It is an object of the invention to provide a method for reconstructing aortic blood pressure from peripheral blood pressure waveform which is accurate for peripheral blood waveforms taken at rest and during exercise of a person under consideration. In particular, it is an object of the invention to model the blood pressure transfer function from suitable heamodynamic parameters derived from the peripheral blood pressure waveform.

To this end the method according to the invention is defined in claim 1 and comprises the steps of:

- determining at least one pre-selected parameter of the peripheral blood pressure waveform;
- reconstructing aortic blood pressure waveform from the peripheral blood pressure waveform using a pressure transfer function having at least one adjustable characteristics, wherein said adjustable characteristics is determined using the at least one pre-selected parameter of the peripheral blood pressure waveform.

Based on profound research data it is found that for each individual and for each exercise level a pressure transfer function from aorta to an extremity, for example to a finger, was different. However, it was found that a certain individual parameters characterizing a peripheral blood pressure waveform can be used as suitable adjustors of the adjustable characteristics of the pressure transfer function. Preferably, for the pressure transfer function a filter is selected described by two second order filter sections, for example a filter known from Gizdulich et al "Models of brachial to finger pulse wave distortion and pressure decrement", Cardiovasc Res 33: 698 - 705, 1997. It will be appreciated that other suitable pressure transfer functions known from the art being constructed to mathematically express a relationship between the blood pressure waveform in the aorta and in a peripheral artery may be used.

The pressure transfer function can be constructed in the frequency domain. This implies that in this case one considers a waveform as a sum of a steady part (mean blood pressure) and a pulsatile part that is composed on sine waves for a given frequency. The transfer function thus expresses a relationship between sine waves for a given frequency between aorta and periphery. In general two parameters suffice to describe the relationship: a ratio between amplitudes of the sine waves (TF modulus) and the difference in phase (TF phase). It is found that TF modulus is in fact a variable, which depends on a physiologic condition of an individual and which may undergo substantial change under exercise. The TF modulus may be characterized by a peak resonance frequency Fpeak, which, in accordance with an aspect of the invention may be individualized for improving accuracy of determination of aortic blood pressure waveform from a peripheral blood pressure waveform. Fpeak thus is found to be a suitable adjustable parameter of the pressure transfer function.

It is found that by deducing a suitable heamodynamic parameter from a peripheral blood pressure waveform the blood pressure transfer function can be accurately modeled in the frequency domain.

It is further found that at least one pre-selected parameter of the blood pressure waveform is identifiable with a reasonable degree of confidence for use as an adjustor of the pressure transfer function. Such at least one pre-selected parameter is cardiac output (CO). The cardiac output (CO) is derived from analysis of the peripheral blood pressure waveform, such as using pulse contour method (pcCO).

It is further found that the resonance peak of the pressure transfer function can be reliably adjusted in accordance with a linear equation:

$$\mathrm{Fpeak = k*CO + const.}$$

Preferably, k = 0.3206, const = 2.6784, the cardiac output being determined using the pulse contour method (pcCO).

k1 = 0.0701;
k2 = 0.0193;
k3 = 0.131;
k4 = 0.0764;
k5 = 0.0234;
const1 = 2.7067;
const2 = 0.6478.

Alternatively but not belonging to the invention, it is possible to use more than one pre-selected parameter for individually adjusting the peak resonance frequency of the pressure transfer function. For example, the resonance peak of the pressure transfer function is adjusted in accordance with an equation:

$$\mathbf{F_{peak} = k1*(MAP\text{-}DBP) + k2*HR - k3*Delay + const1,}$$

or

$$F_{peak} = k4*(MAP-DBP) + k5*HR + const2,$$

wherein the following numerical values for the coefficients and the constants may be assigned:

k1 = 0.0701;
k2 = 0.0193;
k3 = 0.131;
k4 = 0.0764;
k5 = 0.0234;
const1 = 2.7067;
const2 = 0.6478.

These coefficients and constants may be determined using regression analysis of a suitable plurality of measured data for developing a formula determining the frequency at which an individual TF has an extremum, for example a peak ($F_{peak}$). The Gizdulich filter was found to be suitably adaptable for allowing for a peak shift in response to any of the above equations. The combination of the thus determined individualized peak frequency applied to a generic adaptable pressure transfer function yields an individualized pressure transfer function TF (indTF). It is found that by using indTF accuracy of reconstruction of the aortic blood pressure waveform is substantially improved with respect to results obtained with generic transfer function (genTF).

In an embodiment of the method according to the invention, a shift in peak resonance frequency ($F_{peak}$) of the individual aorta-peripheral transfer function (TF) is determined during exercise. For this purpose parameters extracted from the peripheral pressure pulse, as mentioned above may be used. In this way a bias and scatter in the reconstructed aorta pulse pressure present in the method known from the art are mitigated thereby improving accuracy of determination of aortic blood pressure waveform from peripheral measurements, in particularly, during exercise.

It will be appreciated that determination of an aortic blood pressure waveform from a peripheral blood pressure may take place off-line, based on previously acquired peripheral blood pressure waveform data. It is possible that such data is made available by means of a suitable Hospital Information System (HIS), via internet, or, alternatively, it may be stored locally, for example in a suitable blood pressure measurement device.

Preferably, the method according to the invention is carried out on-line and comprises, therefore, the step of measuring the peripheral blood pressure waveform. More preferably peripheral blood pressure is carried out non-invasively, for example, using a finger cuff or using applanation tonometry. It will be appreciated that use of an invasive measurement or a measurement on another suitable extremity is contemplated.

In a further embodiment of the method according to the invention the method further comprises a step of calculating a physiological parameter characteristic of a health condition of the person from at least reconstructed aortic blood pressure waveform data.

It is found to be particularly advantageous to use aortic pressure data reconstructed with increased accuracy, particularly under exercise, for determining a suitable physiological parameter characteristic of a health condition of the individual. For example, cardiac workload in exercise or under psychological stress may be determined and a suitable advice may be put forward to the individual.

A device for reconstructing aortic blood pressure waveform from a peripheral blood pressure waveform is defined in claim 8.

Preferably, the device comprises a measuring unit for measuring the peripheral blood pressure waveform. For example, either an invasive system, or a non-invasive system may be used. Suitable example of the latter relates to a finger cuff or applanation tonometry.

More preferably, the device according to the invention comprises a computing unit for calculating a suitable physiological parameter characteristic of a health condition of the individual.

Optionally, the device according to the invention may form part of a suitable health monitoring system or a fitness apparatus.

A computer program product according to the invention comprises instructions for causing a processor to carry out the steps of the method as is set forth with reference to the foregoing.

These and other aspects of the invention will be discussed in further detail with reference to drawings, wherein like reference signs represent like numerals. It will be appreciated that the drawing are provided for illustrative purposes and may not be used for limiting the scope of the appended claims.

BRIEF DESCRIPTION OF THE DRAWINGS

Figure 1 presents schematically an embodiment of a generic pressure transfer function.
Figure 2 presents schematically an embodiment of an adaptive pressure transfer function in accordance with the invention.
Figure 3 presents schematically an embodiment of a device according to the invention.

DETAILED DESCRIPTION OF THE DRAWINGS

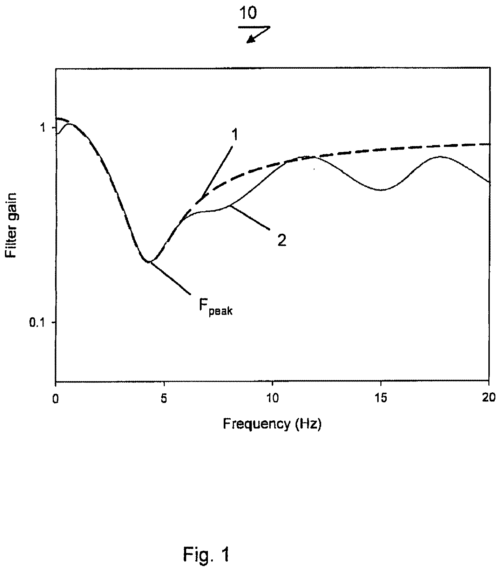

Figure 1 presents schematically an embodiment of a generic pressure transfer function. It will be appreciated that the pressure transfer function 10 relates to a backward pressure transfer function, i.e. a description of a blood pulse transfer from an extremity artery to the aortic artery. In mathematical terms this is the inverse of the more natural forward transfer function. An example of a suitable forward pressure transfer function is known from Gizdulich et al "Models of brachial to finger pulse wave distortion and pressure decrement", Cardiovasc Res 33: 698 - 705, 1997, describing an amplitude transfer of a pressure pulse from aorta to periphery. Accordingly, the transfer function is expressed as follows:

$$H(f) = K \frac{(1 + if/f_0)^2}{1 + 2iDf/f_1 - (f/f_1)^2},$$

wherein

i is the imaginary unit;
K is a gain of the pressure transfer function at 0 Hz,
$f_0$ is a first frequency, $f_0 = f_1 \sqrt{K}$;
f1 is a second frequency;
D is damping.

Figure 1 shows schematically a curve 1, representing a variation of the gain of the pressure transfer function with frequency. It is seen that there is a minimum value of the gain for intermediate frequencies at $F_{peak}$. Curve 2 schematically shows an averaged gain of a multiple transfer functions. Such transfer functions may relate to ARX method, described in Chen CH, Nevo E, Fetics B, Pak PH, Yin FC, Maughan WL and Kass DA: "Estimation of central aortic pressure waveform by mathematical transformation of radial tonometry pressure. Validation of generalized transfer function", Circulation JID - 0147763 95: 1827-1836, 1997.

In order to determine a suitable individualization of a generic transfer function, blood pressure waveform data at rest and at the end of an exercise step may be used. Such data may function as calibration data for determining a suitable relation between an adjustable characteristics of the pressure transfer function, for example the peak frequency $F_{peak}$ and cardiac output derived from a suitable analysis of the pressure waveform, such as pulse contour (pcCO). Preferably, several heart beats of artefact-free continuous data are used for this purpose. It will be appreciated that at least a minimum length of artefact-free data may be one heart beat; and a maximum length of the artefact-free continuous data may be several minutes.

An average ARX transfer function TF (curve 2 in Figure 1) may be determined from the resting data of a suitable plurality of subjects. A transfer function TF described by two 2nd order filter sections, known in the inverse form from Gizdulich et al "Models of brachial to finger pulse wave distortion and pressure decrement", Cardiovasc Res 33: 698 - 705,1997, may then be fitted to the frequency response of the average ARX TF, resulting in a characteristic TF frequency response (curve 1 in Figure 1). This generalized filter (genTF) may be used as a starting point for the individualization.

Based on regression analysis of plurality of patient data a formula was developed that would adjust the frequency at which an individual transfer function TF would peak ($F_{peak}$). Preferably, the Gizdulich filter is adapted to allow for a shift in the peak, which may be implemented using a lookup table.

In order to apply this approach data used for the adjustment of the individual TF peak frequency ($F_{peak}$) may be taken from subject parameters (age, length, weight, BSA), directly available from a peripheral blood pressure wave signal (MAP, DBP, HR, dP/dt) or derived using additional algorithms such as cardiac output derived from an analysis of the pressure waveform, such as pulse contour (pcCO).

It is found that pulse contour cardiac output (pcCO) is a most reliable adjustor for adjusting the peak frequency of the pressure transfer function, particularly during exercise, having a correlation coefficient $R^2 = 0.86$. Correlation coefficients for other pre-selected parameters HR, dP/dt, SV, TPR, delay, estimated PWV, MAP, DBP being 0.71, 0.69, 0.63, 0.63, 0.56, 0.56, 0.51 and 0.35 respectively, may be somewhat lower.

The regression formula for adjustment of the frequency of the peak in the TF from pcCO only may be as follows:

$$F_{peak} = 0.3206*pcCO + 2.6784$$

Multivariate stepwise regression models constructed with the TF peak frequency as the dependent variable resulted in further regression formulas, not part of the invention, with and without pressure wave delay as parameter:

$$F_{peak} = 0.0701*(MAP-DBP) + 0.0193*HR - 0.131*Delay + 2.7067$$

$$F_{peak} = 0.0764*(MAP-DBP) + 0.0234*HR + 0.6478.$$

In accordance with the invention a simple and reliable method is provided for accurately adjusting characteristics of a pressure transfer function for reconstructing aortic blood pressure waveform from a peripheral measurement with an improved accuracy.

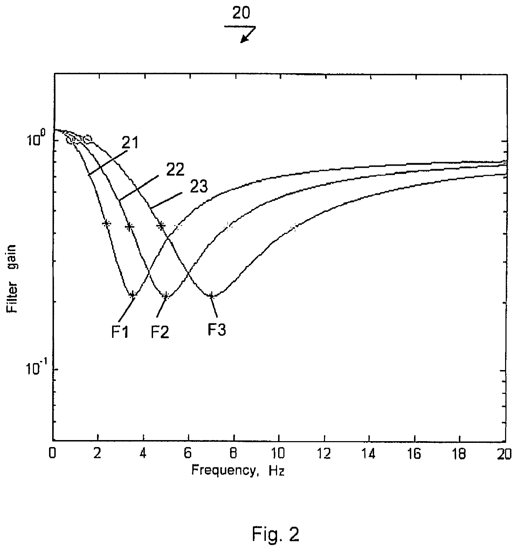

Figure 2 presents schematically an embodiment of an adaptive pressure transfer function in accordance with the invention. The adaptive pressure transfer function 20, may be adjusted yielding actual functions 21, 22, 23 depending on an actual value of a pre-selected parameter, for example cardiac output derived from an analysis of the pressure waveform, such as pulse contour (pcCO). It is seen than for different cardiac outputs, for example during rest or during exercise, different values of the peak frequency F1, F2, F3 of a filter gain are obtained. By applying the thus adjusted pressure transfer function to the peripheral blood pressure waveform aortic blood pressure waveform with improved accuracy may be calculated.

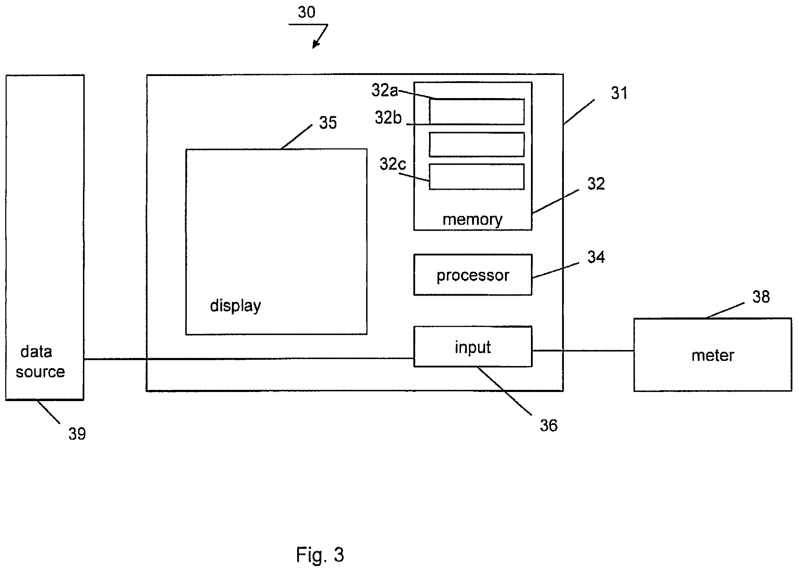

Figure 3 presents schematically an embodiment of a device according to the invention. The device 30 according to the invention comprises a suitable housing 31 comprising an input 36, a processing unit 34 a memory unit 32 and a display 35. The memory unit 32 may be arranged for storing a pressure transfer function 32a having at least one adjustable characteristics and at least a peripheral blood pressure waveform 32b. The processor 34 is preferably arranged for determining at least one pre-selected parameter of the peripheral blood pressure waveform, for determining the adjustable parameter of the pressure transfer function from the at least one pre-selected parameter of the peripheral blood pressure waveform and for reconstructing of aortic blood pressure waveform from a peripheral blood pressure using the pressure transfer function with the adjusted characteristics. For this purpose the memory unit may comprise a computer program 32c arranged for causing the processor 34 to carry out the method of the invention as is described with reference to the foregoing.

Preferably, the device 30 further comprises a measurement unit 38 arranged to measure the peripheral blood pressure waveform, which may relate to either invasive or a non-invasive measuring system. An embodiment of an applicable non-invasive measuring system comprising a finger cuff is known from US 4, 726, 382. The finger cuff may comprise an inflatable bladder formed from a thin flexible, translucent material. The inflatable bladder may be connected to a tube enabling a suitable inflation and deflation of the inflatable bladder. In the finger cuff the inflatable bladder may be the innermost component of the cuff. In order to implement a blood pressure measurement, the finger cuff may comprise a photoplethysmograph.

It will be appreciated that device 30 according to the invention may be arranged to carry out determination of arterial blood pressure waveform on-line. In this case the input 36 may be arranged to receive date (raw or preprocessed) from the measurement unit 38 and to calculate the corresponding aortic blood pressure wave using adjusted pressure transfer function, as described in the foregoing. Display 35 may be arranged to display the measured peripheral blood pressure waveform and the resulting reconstructed aortic blood pressure waveform. Additionally or alternatively, the device 30 may be arranged to continuously measure peripheral blood pressure, eventually out of phase, and to present a corresponding derived arterial blood pressure on the display 35. The arterial blood pressure may be presented as a value. Additionally or alternatively the arterial blood pressure may be presented graphically, for example as a function of time. In an embodiment, such graphic representation may relate to a line indicating a trend in the measured arterial blood pressure.

Alternatively, the device 30 may be arranged to carry out reconstruction of the aortic blood pressure waveform off-line. In this case the input 36 may be arranged to receive suitable data on peripheral blood pressure waveform from a suitable source, like a data carrier, or via intranet or internet.

In a particular embodiment of the device 30 according to the invention, the processor 34 may be arranged to calculate a suitable health-related parameter from the reconstructed aortic pressure waveform data. Preferably, the device 30 forms part of a suitable fitness equipment or a suitable monitoring equipment. More preferable, the calculated health-related parameter is automatically analyzed and a suitable advice to the person is forwarded. The advice may comprise a life style advice, a fitness program advice, or a medical advice. In a particular embodiment the medical advice may relate to a warning or to an advice regarding an increased health hazard leading to a suitable further medical diagnostic advice. In acute circumstances, for example, when the health related parameter signals an impending cardiac overload, the device 30 may be arranged to automatically forward a suitable alarm to dispatch immediate attention of medical personnel. For example a call to a heart alert call center may be automatically realized. This functionality improves para-medical applicability of the device 30 according to the invention.

While specific embodiments have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

**Claims**

1. A method for reconstructing an aortic blood pressure waveform of a person from a peripheral blood pressure waveform of the person comprising the steps of:

   - determining at least one pre-selected parameter of the peripheral blood pressure waveform from analysis of the peripheral blood pressure waveform;
   - reconstructing the aortic blood pressure waveform from the peripheral blood pressure waveform using a pressure transfer function having at least one adjustable characteristic, wherein said adjustable characteristic is determined using the at least one pre-selected parameter of the peripheral blood pressure waveform, wherein the at least one pre-selected parameter of the blood pressure waveform is cardiac output (CO).

2. A method according to claim 1, wherein for the adjustable characteristic a resonance peak of the pressure transfer function is selected.

3. A method according to claim 2, wherein the resonance peak of the pressure transfer function is adjusted in accordance with an equation:

$$Fpeak = k*CO + const.$$

4. A method according to any one of the preceding claims, comprising the step of measuring the peripheral blood pressure waveform.

5. A method according to claim 4, wherein the peripheral blood pressure waveform is measured during an exercise of the person.

6. A method according to any one of the preceding claims 4 or 5, wherein the peripheral blood pressure waveform is measured non-invasively.

7. A method according to any one of the preceding claims, further comprising a step of calculating a physiological parameter characteristic of a health condition of the person from at least aortic blood pressure waveform data.

8. A device for reconstructing an aortic blood pressure waveform from a peripheral blood pressure waveform comprising:

   - a memory unit for storing a pressure transfer function having at least one adjustable characteristic and at least the peripheral blood pressure waveform;
   - a processor arranged for

      - determining at least one pre-selected parameter of the peripheral blood pressure waveform from analysis of the peripheral blood pressure waveform;
      - determining the adjustable parameter of the pressure transfer function from the at least one pre-selected parameter of the peripheral blood pressure waveform;
      - reconstructing of aortic blood pressure waveform from a peripheral blood pressure using the pressure transfer function with the adjusted characteristic,

   wherein the processor is further adapted to select cardiac output (CO) for the at least one pre-selected parameter of the blood pressure waveform.

9. A device according to claim 8, further comprising a measuring unit for measuring the peripheral blood pressure waveform.

10. A device according to claim 9, wherein the measuring unit comprises a finger cuff or applanation tonometry sensor.

11. A computer program product comprising instructions for causing a processor to carry out the steps of the method according to any one of the preceding claims 1 - 7.

**Patentansprüche**

1. Verfahren zur Rekonstruktion einer Wellenform des Aortenblutdrucks einer Person aus einer Wellenform des peripheren Blutdrucks der Person, umfassend die folgenden Schritte:

   - das Bestimmen zumindest eines vorgewählten Parameters der Wellenform des peripheren Blutdrucks aus der Analyse der Wellenform des peripheren Blutdrucks;
   - das Rekonstruieren der Wellenform des Aortenblutdrucks aus der Wellenform des peripheren Blutdrucks mittels einer Druckübertragungsfunktion mit zumindest einer einstellbaren Eigenschaft, wobei die einstellbare Eigenschaft unter Heranziehung des zumindest einen vorgewählten Parameters der Wellenform des peripheren Blutdrucks bestimmt wird, wobei es sich bei dem zumindest einen vorgewählten Parameter der Blutdruckwellenform um das Herzzeitvolumen (HZV, engl. cardiac output, CO) handelt.

2. Verfahren nach Anspruch 1, wobei als einstellbare Eigenschaft eine Resonanzspitze der Druckübertragungsfunktion ausgewählt wird.

3. Verfahren nach Anspruch 2, wobei die Resonanzspitze der Druckübertragungsfunktion gemäß folgender Gleichung eingestellt wird:

$$Fpeak = k*CO + const.$$

4. Verfahren nach einem der vorangehenden Ansprüche, umfassend den Schritt, in dem die Wellenform des peripheren Blutdrucks gemessen wird.

5. Verfahren nach Anspruch 4, wobei die Wellenform des peripheren Blutdrucks während einer körperlichen Belastung der Person gemessen wird.

6. Verfahren nach einem der vorangehenden Ansprüche 4 oder 5, wobei die Wellenform des peripheren Blutdrucks auf nichtinvasive Weise gemessen wird.

7. Verfahren nach einem der vorangehenden Ansprüche, ferner umfassend einen Schritt, in dem ein für eine Erkrankung der Person charakteristischer physiologischer Parameter aus zumindest den Wellenformdaten des Aortenblutdrucks berechnet wird.

8. Vorrichtung zur Rekonstruktion einer Wellenform des Aortenblutdrucks aus einer Wellenform des peripheren Blutdrucks, umfassend:

   - eine Speichereinheit, in der eine Druckübertragungsfunktion mit zumindest einer einstellbaren Eigenschaft sowie zumindest die Wellenform des peripheren Blutdrucks abgespeichert werden;
   - einen Prozessor, eingerichtet zum

     - Bestimmen von zumindest einem vorgewählten Parameter der Wellenform des peripheren Blutdrucks aus der Analyse der Wellenform des peripheren Blutdrucks;
     - Bestimmen des einstellbaren Parameters der Druckübertragungsfunktion aus dem zumindest einen vorgewählten Parameter der Wellenform des peripheren Blutdrucks;
     - Rekonstruieren der Wellenform des Aortenblutdrucks aus einem peripheren Blutdruck unter Heranziehung der Druckübertragungsfunktion mit der eingestellten Eigenschaft,

wobei der Prozessor ferner dafür angepasst ist, als den zumindest einen vorgewählten Parameter der Blutdruckwellenform das Herzzeitvolumen (HZV, engl. cardiac output, CO) auszuwählen.

9. Vorrichtung nach Anspruch 8, ferner umfassend eine Messeinheit, mit der die Wellenform des peripheren Blutdrucks gemessen wird.

10. Vorrichtung nach Anspruch 9, wobei die Messeinheit einen Fingermanschetten-oder einen Applanationstonometriesensor umfasst.

11. Computerprogrammprodukt umfassend Befehle, mit denen ein Prozessor veranlasst wird, die Schritte des Verfahrens gemäß einem der vorangehenden Ansprüche 1-7 auszuführen.

**Revendications**

1. Procédé pour reconstruire une forme d'onde de pression aortique d'une personne à partir d'une forme d'onde de pression périphérique de la personne comprenant les étapes consistant à:

   - déterminer au moins un paramètre présélectionné de la forme d'onde de pression périphérique à partir de l'analyse de la forme d'onde de pression périphérique;
   - reconstruire la forme d'onde de pression aortique à partir de la forme d'onde de pression périphérique en utilisant une fonction de transfert de pression ayant au moins une caractéristique ajustable, dans lequel ladite caractéristique ajustable est déterminée en utilisant le au moins un paramètre présélectionné de la forme d'onde de pression périphérique, dans lequel le au moins un paramètre présélectionné de la forme d'onde de pression de sang est le débit cardiaque (CO).

2. Procédé selon la revendication 1, dans lequel pour la caractéristique ajustable, une crête de résonance de la fonction de transfert de pression est sélectionnée.

3. Procédé selon la revendication 2, dans lequel la crête de résonance de la fonction de transfert de pression est ajustée selon une équation:

$$Fpeak = k*CO + const.$$

4. Procédé selon l'une quelconque des revendications précédentes, comprenant l'étape consistant à mesurer la forme d'onde de pression périphérique.

5. Procédé selon la revendication 4, dans lequel la forme d'onde de pression périphérique est mesurée pendant un exercice de la personne.

6. Procédé selon l'une quelconque des revendications précédentes 4 ou 5, dans lequel la forme d'onde de pression périphérique est mesurée de manière non invasive.

7. Procédé selon l'une quelconque des revendications précédentes, comprenant en outre l'étape consistant à calculer une caractéristique de paramètre physiologique d'un état de santé de la personne à partir au moins de données de forme d'onde de pression aortique.

8. Dispositif pour reconstruire une forme d'onde de pression aortique à partir d'une forme d'onde de pression périphérique comprenant:

   - une unité de mémoire pour mémoriser une fonction de transfert de pression ayant au moins une caractéristique ajustable et au moins la forme d'onde de pression périphérique;
   - un processeur agencé pour

       - déterminer au moins un paramètre présélectionné de la forme d'onde de pression périphérique à partir de l'analyse de la forme d'onde de pression périphérique;
       - déterminer le paramètre ajustable de la fonction de transfert de pression à partir du au moins un paramètre présélectionné de la forme d'onde de pression périphérique;
- reconstruire la forme d'onde de pression aortique à partir d'une pression périphérique en utilisant la fonction de transfert de pression avec la caractéristique ajustée,

dans lequel le processeur est en outre adapté pour sélectionner le débit cardiaque (CO) pour le au moins un paramètre présélectionné de la forme d'onde de pression de sang.

**9.** Dispositif selon la revendication 8, comprenant en outre une unité de mesure pour mesurer la forme d'onde de pression périphérique.

**10.** Dispositif selon la revendication 9, dans lequel l'unité de mesure comprend un doigtier ou un capteur de tonométrie à aplanation.

**11.** Produit de programme informatique comprenant des instructions pour amener un processeur à réaliser les étapes du procédé selon l'une quelconque des revendications précédentes 1 à 7.

Fig. 1

Fig. 2

Fig. 3

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4726382 A


**Non-patent literature cited in the description**

- **STOK et al.** Changes in finger-aorta pressure transfer function during and after exercise. *J Appl Physiology,* 2006, vol. 101, 1207-1214
- **SHARMAN et al.** Validation of a generalized transfer function to noninvasively derived central blood pressure during exercise. *Hypertension,* 2006, vol. 46, 1203-1208
- **GIZDULICH et al.** Models of brachial to finger pulse wave distortion and pressure decrement. *Cardiovasc Res,* 1997, vol. 33, 698-705
- **CHEN CH ; NEVO E ; FETICS B ; PAK PH ; YIN FC ; MAUGHAN WL ; KASS DA.** Estimation of central aortic pressure waveform by mathematical transformation of radial tonometry pressure. Validation of generalized transfer function. *Circulation JID,* 1997, vol. 0147763 (95), 1827-1836
- **GIZDULICH et al.** Models of brachial to finger pulse wave distortion and pressure decrement. *Cardiovasc Res,* 1997, vol. 33, 698-705